# EUROPEAN PATENT APPLICATION

(11) **EP 3 603 496 A1**
(43) Date of publication of application: **05.02.2020**
(21) Application number: 19197501.0
(22) Date of filing: 22.03.2017
(51) Int. Cl.: A61B 5/02, A61B 5/00, G16H 20/40, G16H 50/30, G16H 50/50, G16H 50/70, G16H 30/20, G16H 30/40

(54) **AORTIC ANEURYSM PROGRESSION**

(30) Priority: 08.06.2016 GB 201610040
(62) Divisional of application: 17714532.3
(71) Applicant: Oxford University Innovation Limited, Botley Oxford OX2 0JB (GB)
(72) Inventor: LEE, Regent, Botley, Oxfordshire OX2 0JB (GB); HANDA, Ashok, Botley, Oxfordshire OX2 0JB (GB)
(74) Representative: HGF Limited

(57) **Abstract**

The present disclosure relates to a method for determining a risk factor indicative of predicted growth of an abdominal aortic aneurysm of a patient. The method comprises receiving at least one index of endothelial function of the subject; comparing the or each index of endothelial function with at least one of: a respective reference value; and a further index of endothelial function of the subject; and determining the index of aneurysm future growth based on the comparison. The or each index of endothelial function is indicative of a concentration of one or more circulating biomarkers of endothelial function and a previous aneurysm growth pattern of the subject.

## Description

This invention relates aortic aneurysm progression, in particular to apparatus and a method for determining a risk factor indicative of predicted growth of an abdominal aortic aneurysm of a patient. In particular, the invention may be used to determine the future progression of abdominal aortic aneurysms.

### BACKGROUND

Aortic Aneurysms, in particular Abdominal Aortic Aneurysms (AAAs) are associated with biological changes in the vasculature, features of systemic inflammation and endothelial dysfunction. Rupture of an AAA can result in death in up to 90% of cases. AAAs may present in a subject in a variety of sizes. An AAA is typically defined as a region of an abdominal aortic artery having an outer aortic diameter greater than 30 millimetres. If the outer diameter of the aorta in the region of the aneurysm exceeds 55 millimetres, the abdominal aortic aneurysm is considered to be large. Surgery to treat AAAs is considered where an AAA is identified having a diameter greater than 55 millimetres because the risk of rupture of the AAA is greater than the traditional risks associated with surgery. In current practice, surgery is typically not considered where the identified AAA has a diameter less than 55 millimetres as the risks of surgery are generally considered to outweigh the risk of aneurysm rupture. Currently, where an AAA is identified in a patient, and the AAA has a size between approximately 30 millimetres and 55 millimetres, the size of the AAA is monitored over time. Aneurysms may grow at different rates, if at all. If the aneurysm grows to exceed 55 millimetres, then surgery will typically be performed to treat the aneurysm.

Flow-mediation vasodilation (FMD) is measured following the generation of a vasodilatory stimulus of a distal vascular bed, most commonly the forearm. In practice this may involve the application of a blood pressure cuff on the forearm, which may be inflated to supra peak systolic pressure to generate a short period of blood flow occlusion. The artery (e.g. brachial artery) may be imaged continuously using ultrasound to detect changes in arterial diameter induced by shear stress from changes in blood flow. This test may be coupled with pharmacological supplementation of nitroglycerin to assess the endothelium independent vasodilatory response, which serves as a comparator for FMD mediated through the endogenous release of nitric oxide. The process of occluding the artery (e.g. brachial artery) induces a low flow state, and can lead to constriction of the artery called flow mediated constriction (FMC). This is thought to reflect the basal vaso-constrictive tone conferred by the release of endothelin.

The present disclosure provides at least an alternative to the methods of analysing aneurysms of the prior art by means of predicting the growth of an AAA using a FMD value of a patient.

### BRIEF DESCRIPTION OF THE DRAWINGS

Embodiments of the invention are further described, by way of example, with reference to the accompanying drawings, in which:
Figure 1A is a graph showing a percentage FMD plotted against a diameter of an AAA for each of 162 patients;
Figure 1B is a graph showing a subset of the values shown in Figure 1A;
Figure 2A is a graph showing a future AAA growth rate plotted against AAA diameter for an AAA diameter range of 30 to 55 millimetres;
Figure 2B is a graph showing a future AAA growth rate plotted against AAA diameter for an AAA diameter range of 40 to 55 millimetres;
Figure 2C is a graph showing a future AAA growth rate plotted against AAA diameter for an AAA diameter range of 30 to 39 millimetres
Figure 2D is a graph showing a future AAA growth rate plotted against a percentage FMD for an AAA diameter range of 30 to 55 millimetres;
Figure 2E is a graph showing a future AAA growth rate plotted against a percentage FMD for a AAA diameter range of 40 to 55 millimetres;
Figure 3 shows the relationship between FMD and AAA progression, and the relationship between FMD and AAA surgery;
Figure 4 is a diagram showing an apparatus in accordance with an example of the present disclosure;
Figure 5 is a flow diagram illustrating a method in accordance with an example of the present disclosure.
Figure 6 is a flow diagram illustrating a method in accordance with an example of the present disclosure.

### DETAILED DESCRIPTION

In accordance with the present invention there is provided a method for determining a risk factor indicative of predicted growth of an abdominal aortic aneurysm of a patient. The method comprises receiving a value representative of a size of the abdominal aortic artery; and a flow-mediated vasodilation or flow-mediated constriction value of an artery of the patient (hereinafter "FMD/C value"). The method also comprises determining a risk factor indicative of predicted growth by evaluating the received values with those in an aneurysm risk model. The aneurysm risk model relates to a risk value indicative of predicted growth of an abdominal aortic aneurysm for a given value representative of the size of an abdominal aortic artery and a given FMD/C value.

In current practice, the size of a patient's abdominal aortic aneurysm is used to guide a decision on whether or not the abdominal aortic aneurysm should be surgically removed. As mentioned above, surgical intervention is recommended once the aneurysm reaches a surgical threshold size of, for example, 55 mm. Below this threshold, the risks of surgery are generally considered to outweigh the benefits of aneurysm removal.

It has been found that this prior approach has intrinsic shortcomings because aneurysm size may not an absolute predictor of the risk of rupture. Furthermore, the rate of AAA progression may vary significantly between individuals. For example, there may be a risk of rupture in those individuals with relatively smaller AAAs (e.g. 35 - 55mm or 40-55mm), and many patients with an initially small/moderate size AAA may progress and require surgery within 5 years. This may be an important consideration in ageing populations, as the risks associated with undergoing interventions increase with age.

In the method of the present disclosure, the future growth rate of an individual patient's abdominal aortic aneurysm may be predicted on the basis of the size of a patient's abdominal aortic aneurysm and a flow-mediated vasodilation or flow-mediated constriction value of an artery of the patient. Patients with abdominal aortic aneurysms that are small (e.g. 30 -39 mm) in size tend to have higher FMD values than patients with abnormal aortic aneurysms that are large (e.g. greater than 55 mm). However, for patients with abnormal aortic aneurysms that are small (30 to 39 mm) or medium (e.g. 40 to 55 mm) in size, there is no significant correlation between FMD/C and size.

It has now been found that the future growth rate of an individual patient's abdominal aortic aneurysm can be predicted from the size of a patient's abdominal aortic aneurysm and a flow-mediated vasodilation or flow-mediated constriction value (FMD/C value) of an artery of the patient. This correlation is particularly significant where the size of the abdominal aortic aneurysm is medium in size, for example, in the range of 35 to 55 mm or 40 to 55 mm. This is surprising since no significant correlation between FMD/C and size can be observed in these size ranges. Thus, in a preferred embodiment, the method is used to determine a risk factor indicative of predicted growth of an abdominal aortic aneurysm of a patient identified as having an abdominal aortic artery that is less than 55mm, for example, 30 to 55mm, preferably 35 to 55 mm, more preferably 40 to 55 mm in size. Thus, the method may comprise receiving a value representative of a size of the abdominal aortic artery, wherein the size of the abdominal aortic artery is less than 55mm, for example, 30 to 55 mm or, preferably, 35 to 55 mm or 40 to 55 mm. The subgroup of patients with AAA size between 35 and 55 mm or preferably 40 and 55mm may be particularly important in terms of potential for change in clinical practice. Surgeons may be convinced to provide surgical intervention in this subgroup (e.g. 35 - 40 mm or 40 - 55mm) of patients when there is sufficient justification based, for example, on predicted growth rate.

The method of the present invention may further comprise guiding a decision on a clinical intervention of the patient based on the determined risk factor. For example, if the determined risk factor of a patient is high, the patient's aneurysm may be fast-growing. Thus, the patient in question may benefit from surgery even though his/her aneurysm is below a surgical threshold (e.g. 55 mm) size at which surgery is usually performed. The method of the present invention may be used to identify patients who may benefit from early invasive clinical intervention, for example, even before the patient's aneurysm reaches a surgical threshold (e.g. 55 mm) size. In some embodiments, the clinical intervention may be an invasive clinical intervention, for example, if the size of the abdominal aortic artery is below a surgical threshold, for instance, from 40 to 55 millimetres. Thus, the method of the present invention can be used to negate unnecessary delay in surgery (as those patients with fast growing AAAs would require surgery in the near future).

The method of the present invention may also be used to guide a decision on a clinical intervention that is non-invasive or cognitive. For example, where the determined risk factor of a patient is low, the patient's aneurysm may be slow-growing. Thus, the patient in question need not be monitored highly frequently even, for example, if the patient's aneurysm is relatively close to the threshold size (e.g. 55 mm) at which surgery is usually performed. Accordingly, a schedule e.g. for the patient's future check-ups may be determined at least in part by the determined risk factor. Thus, in some embodiments, the method of the present invention may be used to guide a physician's decision as to, for example, the time interval between the patient's follow-up appointment(s). In some embodiments, this surveillance or time interval may be individualised to the patient based on the predicted propensity of future AAA growth.

In one embodiment, the method comprises receiving a value representative of a size of the abdominal aortic artery; and a flow mediated dilatation value (hereinafter "FMD value") of an artery of the patient. The FMD value may be the FMD value of the brachial artery of a patient. In a preferred embodiment, the method further comprises measuring the FMD value at the brachial artery of the patient. Any suitable method for measuring FMD may be employed. For example, the FMD value may be measured by (e.g. continuously) imaging the brachial artery using ultrasound. FMD can also be measured in other arteries such as the femoral (thigh) artery.

The method may also comprise measuring the size of the abdominal aortic artery of a patient. Any suitable method of measurement may be employed. For example, the maximal anteroposterior diameter (outer-to-outer) of an abdominal aortic artery may be determined by, for example, by ultrasound scans, computerised tomography, or magnetic resonance imaging.

In some embodiments, the aneurysm risk model is a look-up table. The aneurysm risk model may be generated based on: FMD/C measurements and the size of an abdominal aortic artery of at least one patient taken at a first time; and FMD/C measurements and the size of an abdominal aortic artery of the at least one patient taken at a second time different from the first time. The aneurysm risk model may also generated on the basis of the value representative of a size of the abdominal aortic artery at the first time and the size of the abdominal aortic artery at the second time. In some examples, the aneurysm risk model is generated from data collected from a number of patients e.g. in a clinical trial.

The risk factor indicative of predicted growth of an abdominal aortic aneurysm of a patient may be a qualitative (e.g. fast, medium or slow) or quantitative (e.g. mm per unit time) measurement.

As well as FMD/C measurements, the method may also comprise receiving other inputs indicative of the endothelial function of the subject. An example of such an input may be the concentration of circulating biomarkers of endothelial function in the patient's blood. Such circulating biomarkers of endothelial function may be one or more of: an endothelin protein, a relaxin protein, a plurality of proteins, genomes and/or metabolites. In one embodiment, the circulating protein and/or metabolite profile may comprise a number, for example, over 50 proteins and/or metabolites. The method may further comprise determining a risk factor for aneurysm growth from a circulating protein and/or metabolite profile, in combination with aneurysm size and FMD/C of a patient's artery.

In one embodiment, the present disclosure provides a method for determining a risk factor indicative of predicted growth of an abdominal aortic aneurysm of a patient having an abdominal aortic aneurysm that is less than 55 mm, preferably 30 to 55 mm, more preferably 35 to 55 mm, yet more preferably 40 to 55 mm in size. The method comprises receiving a value representative of a size of the abdominal aortic artery, wherein the received value is representative of an abdominal aortic artery that is less than 55 mm, preferably 30 to 55 mm, more preferably 35 to 55 mm, yet more preferably 40 to 55 mm in size. The method also comprises receiving a FMD/C value. The method further comprises determining a risk factor indicative of predicted growth by evaluating the received values with those in an aneurysm risk model. The aneurysm risk model relates to a risk value indicative of predicted growth of an abdominal aortic aneurysm for a given value representative of the size of an abdominal aortic artery and a given FMD/C value. The given value representative of the size of an abdominal aortic artery of the risk model may be a value that is representative of an abdominal aortic artery that is less than 55 mm, preferably 30 to 55 mm, more preferably 35 to 55 mm, yet more preferably 40 to 55 mm in size.

In some examples, the method can be used to determine a risk factor indicative of predicted growth of an abdominal aortic aneurysm of a patient having an abdominal aortic aneurysm that is 35 to 55 mm in size, preferably 36 to 53 mm, more preferably 38 to 50 mm in size. The method may involve receiving a value representative of a size of the abdominal aortic artery, wherein the received value is representative of an abdominal aortic artery that is 35 to 55 mm in size, preferably 36 to 53 mm, more preferably 38 to 50 mm in size. The given value representative of the size of an abdominal aortic artery of the risk model may be a value that is representative of an abdominal aortic artery that is 35 to 55 mm in size, preferably 36 to 53 mm, more preferably 38 to 50 mm in size.

In accordance with a further aspect of the present invention, there is provided an apparatus for determining a risk factor indicative of predicted growth of an abdominal aortic aneurysm of a patient. The apparatus comprises a data input to receive a value representative of a size of an abdominal aortic artery of a patient; and a flow-mediated vasodilation or flow-mediated constriction value (FMD/C value) of an artery of the patient. The apparatus also comprises at least one processor; and a memory comprising an aneurysm risk model relating to a risk value indicative of predicted growth of an abdominal aortic aneurysm for a given value representative of the size of an abdominal aortic artery and a given FMD/C value. The memory also comprises instructions executable by the at least one processor to cause the processor to retrieve from the aneurysm risk model, using the received data inputs, a risk factor indicative of predicted growth of an abdominal aortic aneurysm.

In the apparatus described herein, the data input may receive a value representative of a size of the abdominal aortic artery; and an FMD value. The FMD value may be a brachial FMD value.

In some embodiments, the data input may be a user input device such as a touchscreen interface or a keypad. The data input may be a removable memory connector to receive a removable storage medium to store a value representative of a size of an abdominal aortic artery of a patient; and a flow-mediated vasodilation or flow-mediated constriction value of an artery of the patient. The data input may be a wired or a wireless data connection to a sensor device configured to sense, or determine, based on sensed data, a value representative of a size of an abdominal aortic artery of a patient; and/or an FMD/C value of an artery of the patient.

In some embodiments, the apparatus may further comprise a sensor device in data communication with the data input and configured to measure the FMD value at the brachial artery of the patient. The sensor device may measure the FMD value by means of (e.g. continuously) imaging the brachial artery using ultrasound.

Where the apparatus comprises a data memory in data communication with the data input, the data memory may be configured to store one or more of the at least one value representative of a size of an abdominal aortic artery of a patient; and a FMD/C value of the patient. The memory may comprise instructions to cause the processor to read the one or more of the value representative of a size of an abdominal aortic artery of a patient; and a FMD/C value of the patient from the data memory. The memory may further comprise instructions to cause the processor to measure the FMD/C value or size of the abdominal aortic artery.

In yet another aspect, the present invention provides a non-transitory machine-readable storage medium encoded with instructions executable by a processor for determining a risk factor indicative of predicted growth of an abdominal aortic aneurysm of a patient. The machine-readable storage medium comprises instructions to receive a value representative of a size of the abdominal aortic artery; and a flow-mediated vasodilation or flow-mediated constriction value (FMD/C value) of the patient. The machine-readable storage medium also comprises instructions to determine the risk factor indicative of predicted growth by evaluating the received values with those in an aneurysm risk model, the aneurysm risk model relating to a risk value indicative of predicted growth of an abdominal aortic aneurysm for a given value representative of the size of an abdominal aortic artery and a given FMD/C value

The non-transitory machine-readable storage medium may further comprise instructions to receive a value representative of a size of the abdominal aortic artery; and a flow-mediated vasodilation value (FMD/C value) of an artery of the patient. The FMD value may be an FMD value of a brachial artery of the patient.

The non-transitory machine-readable storage medium may further comprise instructions to measure the FMD value at the brachial artery of the patient.

The non-transitory machine-readable storage medium may further comprise instructions to measure the FMD value by means of continuously imaging the brachial artery using ultrasound.

The non-transitory machine-readable storage medium may further comprise an aneurysm risk model in the form of a look-up table.

In yet a further aspect, there is provided an apparatus for determining an index of aortic aneurysm future growth for a subject. The apparatus comprises a data input for receiving at least one index of endothelial function of a subject, at least one processor, and a memory comprising instructions executable by the at least one processor. The instructions are to cause the at least one processor to compare the or each index of endothelial function with at least one of a respective reference value and a further index of endothelial function for the subject. The instructions are further to cause the processor to determine the index of aortic aneurysm future growth based on the comparison. The method may determine an index of abdominal aortic aneurysm future growth. It will be understood that the future growth of the aortic aneurysm is a determination of the future progression of the aortic aneurysm.

Thus, there is provided apparatus for determining an index of aneurysm future progression (e.g. growth) for a subject. The apparatus may be used on subjects regardless of knowledge of the presence of an aneurysm in the subject. In many examples, the apparatus may be used on subjects where the presence and size of an aneurysm are already known. It will be understood that an index of endothelial function is any value of an indicator of the function of an endothelium (the inner lining of blood vessels). The index of endothelial function may not be derived from measurements solely directed to a region of a blood vessel comprising an aneurysm. An index type of the index of endothelial function is the particular type of indicator of the function of the endothelium.

The index of aneurysm future progression may be qualitative or quantitative.

One or more of the at least one index of endothelial function may be indicative of a flow-mediated vasodilation value. One or more of the at least one index of endothelial function may be indicative of a flow-mediated constriction value. One or more of the at least one index of endothelial function may be indicative of a concentration of one or more circulating biomarkers of endothelial function of the subject. One or more of the at least one index of endothelial function may be based on a previous aneurysm growth pattern of the subject. In embodiments, the or each index of endothelial function may be indicative of at least one of: the flow mediated vasodilation value; the flow-mediated constriction value; aneurysm size, the concentration of one or more circulating biomarkers of endothelial function of the subject; and the previous aneurysm growth pattern of the subject. In embodiments, the or each index of endothelial function may be indicative of at least one of: the flow mediated vasodilation value or the flow-mediated constriction value; aneurysm size and optionally additionally the concentration of one or more circulating biomarkers of endothelial function of the subject; and the previous aneurysm growth pattern of the subject.

The circulating biomarkers of endothelial function may be one or more of: an endothelin protein, a relaxin protein, a plurality of proteins, genomes and/or metabolites, whereby to form a circulating protein, genomic and/or metabolite profile. In one embodiment, the circulating protein and/or metabolite profile may comprise over a number e.g. over 50 proteins and/or metabolites. The method may further comprise determining the index of endothelial function from the circulating protein and/or metabolite profile. The method may comprise determining the index of endothelial function from the genomic profile. The endothelin protein may be a plurality of endothelin proteins. The relaxin protein may be a plurality of relaxin proteins.

The data input may be a user input device such as a touchscreen interface or a keypad. The data input may be a removable memory connector to receive a removable storage medium to store the at least one index of endothelial function of the subject. The data input may be a wired or wireless data connection to a sensor device configured to sense, or determine based on sensed data, the at least one index of endothelial function. In examples, the data input may be provided by a combination of the options described previously. The sensor device may comprise a sensor for determining FMD/C and at least one of a biomarker estimation or measurement module, a blood pressure estimation module, for example in the form of a blood pressure cuff or may comprise a blood sample analysis module configured to analyse a blood sample, or a sample derived therefrom, to determine the presence or concentration of one or more predetermined circulating biomarkers for endothelial function.

The apparatus may further comprise a data memory in data communication with the data input. The data memory may be configured to store one or more of the at least one index of endothelial function. The memory may comprise instructions to cause the processor to read the one or more of the at least one index of endothelial function from the data memory.

The memory may further comprise instructions to cause the processor to determine one or more of the at least one index of endothelial function by controlling a module to measure the flow-mediated vasodilation value. The memory may further comprise instructions to cause the processor to determine one or more of the at least one index of endothelial function by controlling a module to measure the flow-mediated constriction value. The memory may further comprise instructions to cause the processor to determine one or more of the at least one index of endothelial function by controlling the blood sample analysis module to measure the concentration of one or more circulating biomarkers of endothelial function of the subject.

One or more of the at least one index of endothelial function may be based on measurements taken at a first time. The further index of endothelial function may be based on measurements taken at a second time. The second time may be different from the first time. Thus, a time-spaced set of indices of endothelial function can be used to determine an index of aneurysm future growth. In this way, information about a change in a subject's endothelial function over time may be determined and used to determine information about a potential growth rate of an aneurysm. The second time may be later than the first time. The second time may be over one week later than the first time. The second time may be over one month later than the first time. The second time may be over one year later than the first time. The second time may be over six months later than the first time.

In some embodiments, an index type of one or more of the at least one index of endothelial function may be different from an index type of the further index of endothelial function. For example, the memory may comprise instructions to cause the processor to compare an indicator of a flow-mediated vasodilation value with one or more different metrics of endothelial function. For example, the memory may comprise instructions to cause the processor to compare an indicator of a flow-mediated vasodilation (or constriction) value with an indicator of a concentration of one or more circulating biomarkers of endothelial function of the subject. In examples, the further index of endothelial function may be a further one of the at least one index of endothelial function. In some embodiments an index type of one or more of the at least one index of endothelial function may be the same as an index type of the further index of endothelial function. For example, one or more of the at least one index and the further index may both be indicative of a flow-mediated vasodilation value.

The reference value may be an average or expected value for the index of endothelial function.

When the one or more of the at least one index of endothelial function is indicative of the concentration of one or more circulating biomarkers, the memory may further comprise instructions to cause the processor to analyse, in vitro using a blood sample analysis module, a sample derived from a blood sample of the subject. The memory may further comprise instructions to cause the processor to determine the one or more of the at least one index of endothelial function based on the analysis of the sample.

When the one or more of the at least one index of endothelial function is indicative of a flow-mediated vasodilation value, the memory may further comprise instructions to cause the processor to determine a dilatation value indicative of a vasodilation of a blood vessel as a result of a change (e.g., an increase) in fluid flow rate through the blood vessel. The memory may further comprise instructions to cause the processor to determine the one or more of that at least one index of endothelial function based on the determination of the dilatation value.

When the one or more of the at least one index of endothelial function is indicative of a flow-mediated constriction value, the memory may further comprise instructions to cause the processor to determine a constriction value indicative of a constriction of a blood vessel as a result of a change (e.g., a decrease) in fluid flow rate through the blood vessel. The memory may further comprise instructions to cause the processor to determine the one or more of the at least one index of endothelial function based on the determination of the constriction value.

The memory may further comprise instructions to cause the processor to store the index of aneurysm future growth in the memory. The memory may further comprise instructions to cause the processor to store the index of aneurysm future growth in a further memory.

Viewed from another aspect, there is provided a method for determining an index of aneurysm future growth for a subject. The method comprises receiving at least one index of endothelial function of the subject, comparing the or each index of endothelial function with at least one of a respective reference value and a further index of endothelial function of the subject, and determining the index of aneurysm future growth based on the comparison.

Viewed from a further aspect, there is provided a computer-implemented method for determining an index of aneurysm future growth for a subject. The method is implemented on a computer comprising at least one processor and a memory comprising instructions to be executed by the at least one processor. The method comprises: the memory of the computer receiving at least one index of endothelial function of the subject. The method further comprises the at least one processor comparing the index of endothelial function with at least one of a respective reference value and a further index of endothelial function of the subject. The method further comprises the at least one processor determining the index of aneurysm future growth based on the comparison.

The computer-implemented method may further comprise storing index of aneurysm future growth in the memory or a further memory.

The invention extends to a non-transitory machine-readable storage medium encoded with instructions executable by a processor. The machine-readable storage medium comprises instructions to receive at least one index of endothelial function of a subject. The machine-readable storage medium further comprises instructions to compare the index of endothelial function with at least one of a respective reference value and a further index of endothelial function of the subject. The machine-readable storage medium further comprises instructions to determine an index of aneurysm future growth based on the comparison.

It will be understood that the present disclosure extends to any of the steps of a method performed by the apparatus disclosed herein.

Aspects of the present invention will now be described in further detail with reference to the accompanying drawings.

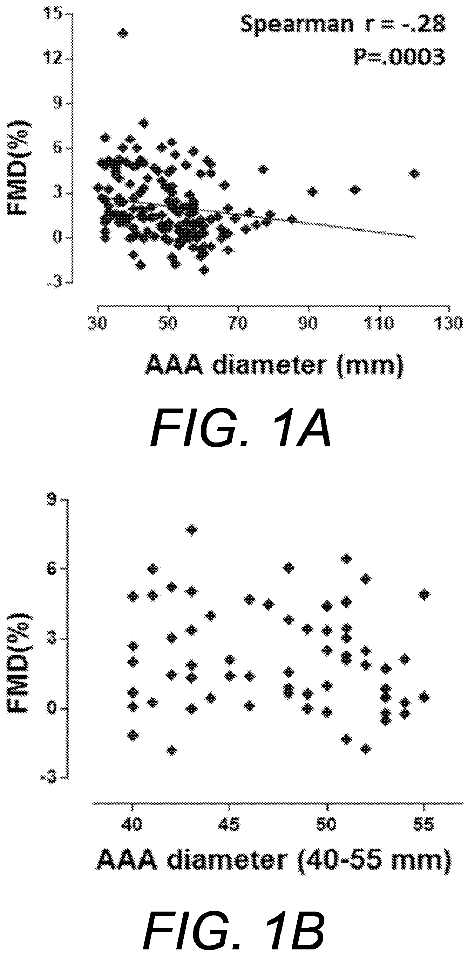

Flow mediated dilatation (FMD) is a mainstream method for non-invasive assessments of systemic endothelial function. Flow mediated dilatation is a measure of the percentage increase of a diameter of an artery in response to an increase in the flow of blood through the artery after constriction of the flow of blood e.g. using a blood pressure cuff. Changes in FMD is typically denoted by percentage change of FMD (FMD%). It has previously been found that there is an association between FMD and the size of AAAs. Figure 1A is a graph showing the percentage FMD plotted against a diameter of an AAA for 162 patients having an age range between 51 and 92. The FMD% values range from approximately -2% to 14%. The AAA size measurements range from approximately 30 millimetres to 120 millimetres. The data shows an inverse correlation between the FMD% and the size of the AAA. In other words, patients having a larger AAA are more likely to demonstrate a lower FMD% value. Figure 1B is a graph showing a subset of the data shown in Figure 1A (dotted line zone). In particular, the graph shown in Figure 1B is restricted to only relate to AAAs having a diameter between 40 millimetres and 55 millimetres. In this subset of data shown in Figure 1B, there is no correlation between the FMD% value and the diameter of the AAA.

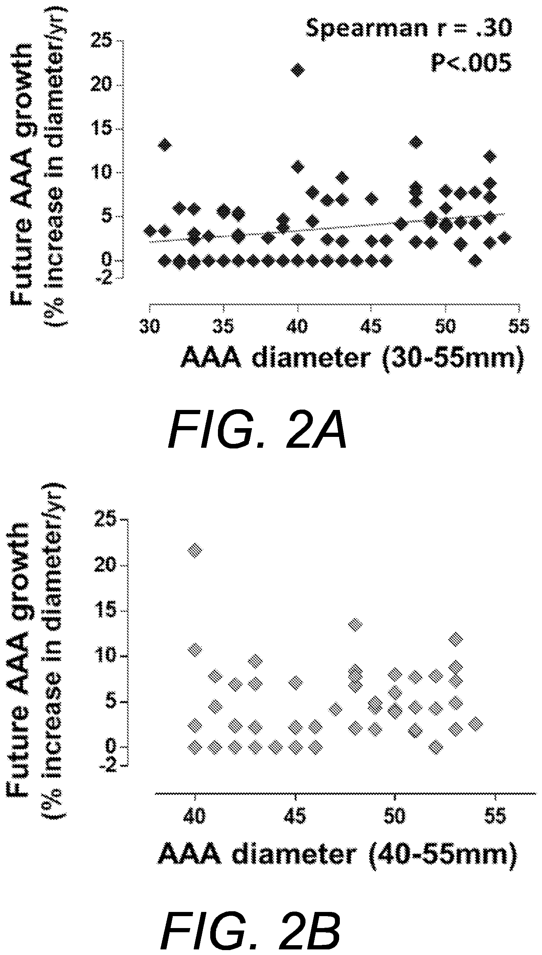

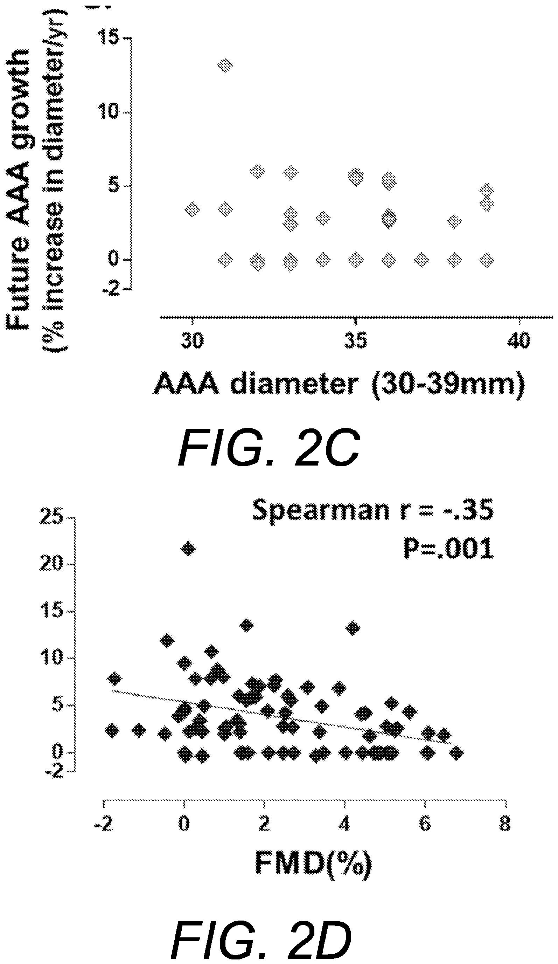

Figures 2A, 2B and 2C are graphs showing future AAA growth rate plotted against AAA diameter taken at baseline. These data points are derived from the same dataset as used to produce the graphs shown in Figures 1A and 1B. A first diameter of the aneurysm in each patient was measured and recorded at this first measurement time. A second diameter of the aneurysm in each patient was measured and recorded at a second measurement time. The second measurement time was approximately 1 year after the first measurement time The median duration of follow-up for this group was 365 days (interquartile range: 343 to 381 days). The observed growth rate of the aneurysm in %increase in diameter/ year could then be determined using the first diameter, the first measurement time (including date), the second diameter and the second measurement time (including date).

Figure 2A shows that there was an overall correlation between the baseline diameter of AAAs and the growth rate in the future 12 months. However, when the participants were further stratified according to AAA diameter (30-39mm, 40-55mm), the median growth rate over the following 12-month period differed significantly between the small AAAs (30-39mm) and the moderate AAAs (40-55mm) (2.6% vs 4.2%, Mann-Whitney test P<.01). Further examination of the AAA subgroups showed no correlation between baseline diameter vs the future growth rate in either the moderate size AAAs (40-55mm) (Figure 2-B, Spearman r = .12, P=.39) or in the subgroup of small size AAAs (30-39mm (Figure 2-C, Spearman r = -.02, P =.9). Thus, for small and medium AAAs (30-39mm, 40-55mm), there was little correlation between aneurysm size and future growth.

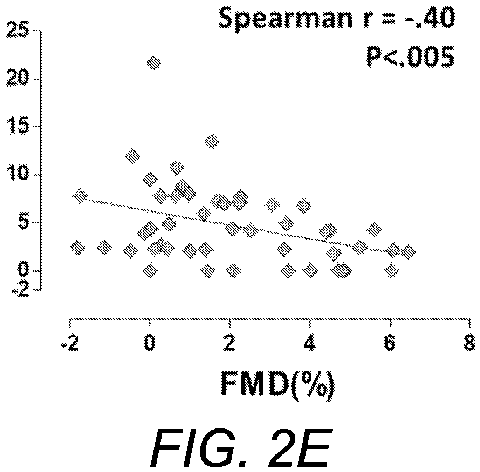

Figure 2D and 2E are graphs showing future AAA growth rate plotted against a percentage FMD taken at baseline. These data points are derived from the same dataset as used to produce the graphs shown in Figures 1A and 1B. A first diameter of the aneurysm in each patient was measured and recorded at this first measurement time. A second diameter of the aneurysm in each patient was measured and recorded at a second measurement time. The second measurement time was approximately 1 year after the first measurement time The median duration of follow-up for this group was 365 days (interquartile range: 343 to 381 days). The observed growth rate of the aneurysm in %increase in diameter/ year could then be determined using the first diameter, the first measurement time (including date), the second diameter and the second measurement time (including date).

Figure 2D includes patients with AAA diameters between 30-55mm. It shows an inverse correlation between the FMD% value measured at the first measurement time and the growth rate of the aneurysm between the first measurement time and the second measurement time. Figure 2E includes the subgroup of patients with AAA diameter between 40-55mm. The strength of association in this subset is stronger (as signified by the Spearman rho coefficient). This further highlights the importance of the FMD observation in the clinically relevant subgroup of AAAs with diameter between 40-55mm. The graphs shown in Figure 2D and 2E support the finding of a relationship between future progression of aneurysms (in particular aortic aneurysms, more particularly abdominal aortic aneurysms) and FMD. Using this dataset, a mathematical method can be constructed for the prediction of future 12-month growth of an AAA in an individual. As an example, the receiver operation characteristics (ROC) analysis for prediction of AAA growth <1 mm/year by this method shows that the area under ROC curve (AUROC) is 80%, with the following statistics: specificity 94%, positive predictive value (PPV) 80%, negative predictive value (NPV) 78%. As another example, the AUROC for the prediction of AAA growth ≥3mm/year is 67%, with the following statistics: specificity 86%, PPV 38%, NPV 71%.

This data supports the use of e.g. FMD, to identify patients who, should they have an aneurysm (in particular an aortic aneurysm, more particularly an abdominal aortic aneurysm), may have a slow or fast growing aneurysm, for which further monitoring may be necessary. In some cases, a physician may use aneurysm future growth information, in conjunction with other patient information, to assess a patient's potential risk-benefit profile to determine the frequency of monitoring or timing of a surgical procedure to treat the aneurysm.

Although the data presented in Figure 2D and 2E shows a relationship between FMD and aneurysm future growth, it will be understood that the data may potentially indicate plausibility for a relationship between other types of index of endothelial function and aneurysm future progression. Other index types may be used in combination with FMD/C to predict future aneurysm future growth. Example of such other index types include values of flow mediated constriction, concentrations (either alone or in combination) of one or more circulating biomarkers of endothelial function, and others. It will also be understood that combining multiple index types of the index of endothelial function for use in determining an index of aneurysm future progression may lead to a more accurate determination of the index of aneurysm future progression. The index of aneurysm future progression may be qualitative or quantitative.

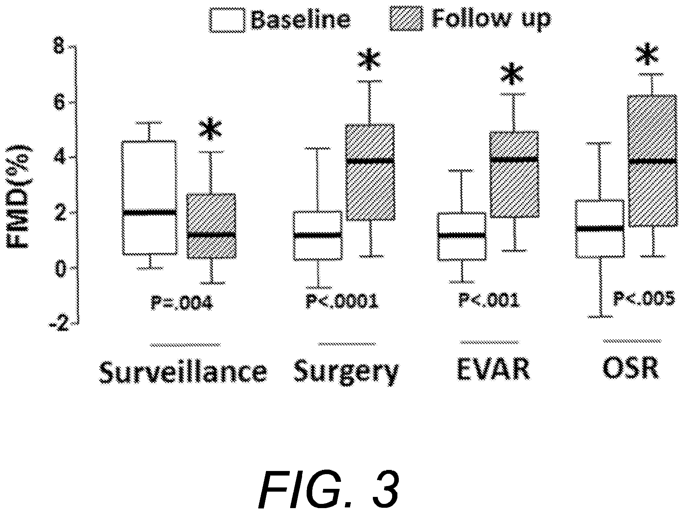

Figure 3 shows the relationship between FMD and AAA progression, and FMD and surgery. FMD reduced significantly between assessments during AAA surveillance (from a median of 2.0% at baseline to 1.2% at follow up, Wilcoxon matched pairs signed rank test [WMPST] P=.004). FMD was measured in 50 patients prior to AAA surgery, and remeasured at 8-12 weeks after. FMD improved significantly after surgical repair (from 1.1% pre-op to 3.8% post-op, WMPST P<.0001), irrespective of the type of surgery performed (EVAR n=28; open surgical repair n=22).

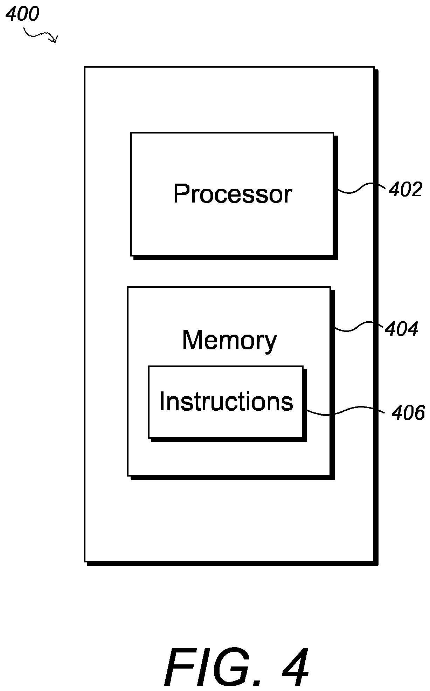

Figure 4 is a diagram showing an apparatus in accordance with an example of the present disclosure. The apparatus 400 comprises a processor 402 and a memory 404. An instruction region 406 of the memory 404 comprises instructions to cause the processor to carry out steps of the method described herein. The apparatus 400 may further comprise a sensor for measuring FMD/C (not shown) or a blood pressure estimation module(biomarker estimation module) (not shown), for example in the form of a blood pressure cuff, and optionally a blood sample analysis module (not shown). In particular, the apparatus 400 is for use in determining a risk factor indicative of predicted growth of an abdominal aortic aneurysm of a patient based on a value representative of a size of the abdominal aortic artery and a FMD/C value of an artery of the patient.

It will be appreciated that examples described herein can be realised in the form of hardware, or a combination of hardware and software. Any such software may be stored in the form of volatile or non-volatile storage such as, for example, a storage device like a ROM, whether erasable or rewritable or not, or in the form of memory such as, for example, RAM, memory chips, device or integrated circuits or on an optically or magnetically readable medium such as, for example, a CD, DVD, magnetic disk or magnetic tape. It will be appreciated that the storage devices and storage media are examples of non-transitory machine-readable storage that are suitable for storing a program or programs that, when executed, implement examples described herein. Accordingly, examples provide a program comprising code for implementing a system or method as described herein and a machine readable storage storing such a program.

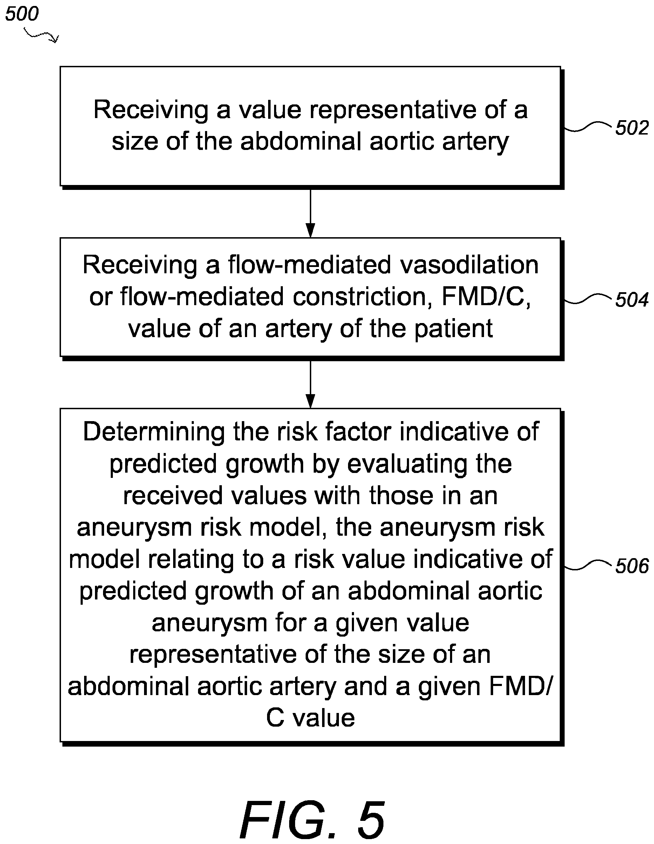

Figure 5 is a flow diagram illustrating a method in accordance with an example of the present disclosure. The method 500 comprises a first method step 502 of receiving a value representative of a size of the abdominal aortic artery. The method next comprises a second method step 504 of receiving a FMD/C value of an artery of the patient. The method next comprises a third method step of determining the risk factor indicative of predicted growth by evaluating the received values with those in an aneurysm risk model, the aneurysm risk model relating to a risk value indicative of predicted growth of an abdominal aortic aneurysm for a given value representative of the size of an abdominal aortic artery and a given FMD/C value. As will be understood the method is performed in the order described herein. In another embodiment the first step 502 and second step may be substituted with one another, or further alternatively performed simultaneously.

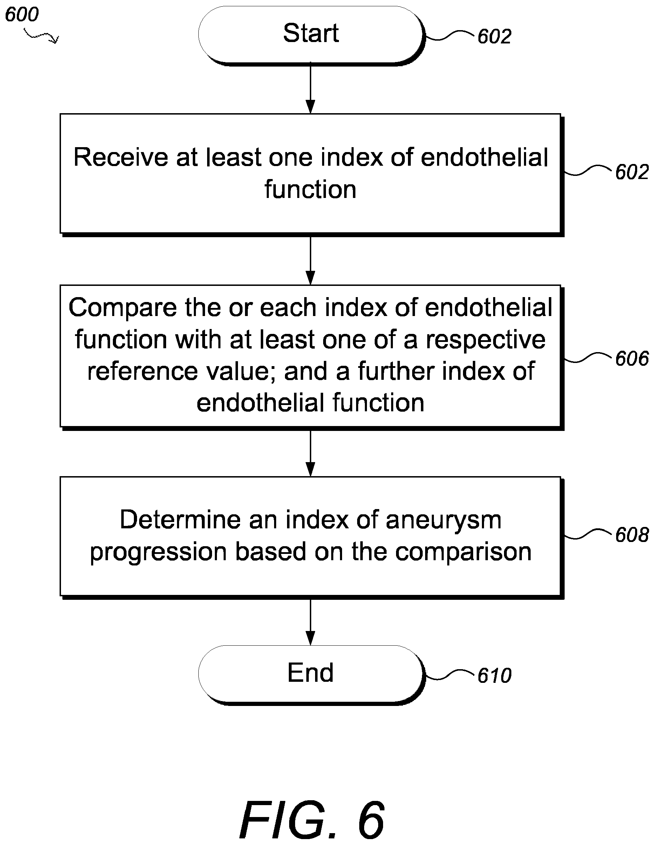

Figure 6 is a flow chart illustrating a method in accordance with an example of the present disclosure. The method 600 comprises a start 602. The method 600 next comprises a first method step 604 of receiving at least one index of endothelial function. The method 600 further comprises a second method step 606, after the first method step 604. The second method step 606 comprises comparing the or each index of endothelial function with at least one of a respective reference value and a further index of endothelial function. The method 600 further comprises a third method step 608 of determining an index of aneurysm progression based on the comparison performed in the second method step 606. The method 600 terminates in a termination step 610, subsequent to the third method step 608. As will be understood, the method 600 is performed in the order described herein.

### Examples

### Methods

Patients with infra-renal AAAs (aortic diameter ≥ 30mm) under surveillance and those that had an incidentally diagnosed AAA requiring surgery were recruited for this study.

### AAA size and subgroups

For each participant, the AAA size was obtained. The AAA size was defined by the maximal anteroposterior diameter (outer-to-outer). For this study, an AAA was defined by anteroposterior diameter (APD) greater than 30mm. Further subgroups were defined according APD into small (30-39mm), moderate (40-55mm), and large (>55cm) AAAs. The annual growth rate of AAA during surveillance was calculated by: (ΔAPD/APD at baseline) / (number-of-days-lapsed/365days).

For the analyses pertaining to future growth rate, focus was placed on AAAs smaller than 55mm at the time of recruitment. Beyond this point, it was reasoned that biomarkers of future AAA progression should have little impact on existing clinical practice as with large AAAs (>55mm) should have been referred for surgery promptly, and those who do not undergo surgery beyond this size are likely to be affected by other confounding factors. The distinction was set between the subgroup of small and moderate size AAAs, as the previous clinical trials on early surgery vs surveillance for AAAs used 40-55mm as the size criteria for inclusion. On the other hand, patients with small AAAs (30-39mm) are unlikely to be offered surgery even in a clinical trial setting.

### Ultrasound image acquisition and analyses

Ultrasound imaging of the brachial artery was then performed to record flow mediated responses. The procedure was performed in a dimly lit quiet room, with the patient in the supine position. ECG tabs were applied to enable ECG gated acquisition of images. Brachial artery in the right arm was identified by ultrasound and the baseline measurement obtained. Supra-systolic pressure was applied to the forearm via the blood pressure cuff for 5 minutes to achieve blood flow occlusion. The diameter of the brachial arterial was recorded at specific time points during flow occlusion and after the release of the blood pressure cuff to assess flow mediated changes in diameter.

Analysis of flow mediated dilatation were performed by assessors blinded to the clinical characteristics of each patient using the Vascular Research Tool (Version 6.7.4, Medical Imaging Applications LLC) with adherence to standardised operating procedures. The percentage change in flow mediated dilatation was calculated by: (Δbrachial artery diameter before and after flow stimuli/brachial artery diameter at baseline). For the calculation of FMD% and NMD%, the maximum diameter of brachial artery during the respective measurement period is used. Although this method is used in these examples, it will be understood that other methods for measuring FMD may be used.

In these examples reproducibility of the FMD analysis was assessed by repeated scans by two different assessors on 10 healthy individuals under the same conditions, with 30 minutes' rest in between scans. Intra-observer variation was assessed by re-analyses of randomly selected scans by the same assessor. The co-efficient of reproducibility and intra-observer variation for FMD analysis were 5% and 6% respectively. Although this method is used in these examples, it will be understood that other methods for measuring the reproducibility of the FMD analysis may be used.

### Statistical analysis

Statistical analyses were performed in Graphpad Prism Version 6.01. Exploratory data analysis was performed for the initial examination of the dataset. Summary statistics are presented in mean (with SD) or median (with IQR) depending on the normality of distribution. We opted to use non-parametric tests for all comparative analyses (Wilcoxon matched pairs signed rank test, Mann-Whitney test, Kruskal-Wallis test, and Spearman rank correlation), as many variables demonstrated non-Gaussian distributions. No transformation of data was performed.

### Results

### Participant characteristics

162 patients with AAAs were recruited (Male n=147; Females n=15). The median AAA diameter was 50mm (IQR: 40-57mm). The average age of participants was 75 (+/-7) years old at the time of consent. The majority were ex-smokers (66%) and 19% were current smokers. A history of symptomatic atherosclerotic arterial disease was prevalent in this group (ischaemic heart disease: 40%; peripheral arterial disease: 20%; cerebral vascular disease: 12%). The majority of participants reported a prior diagnosis of arterial hypertension (65%) and hypercholesterolemia (59%). However, these were well controlled by long term medical therapy [anti-hypertensive(s): 71%, statin: 75%, anti-platelet(s): 62%], as reflected by their controlled SBP/DBP (138/79 ±17/12 mmHg) and overall normal cholesterol profiles [median = 3.9 mmol/L (IQR 3.3-4.7), lower than 5.2 mmol/L in 82% of participants] at the time of recruitment. Seventeen percent of the participants reported a history of diabetes mellitus, 19% had chronic respiratory disease, 19% had treated neoplasms, 24% had chronic kidney disease with eGFR<60.

### Correlation between FMD and the diameter of AAA

Amongst all participants, there was a weak significant inverse correlation between the diameter of AAA and FMD at the baseline assessment (Spearman r = -.28, P=.0003) (Figure 1-A). However, such correlation was no longer observed within the subgroup of moderate size AAAs (40-55mm) (Figure 1-B, Spearman r = -.1, P=.4) or in the small size AAAs (30-39mm) (Spearman r = .04, P=.8, data not shown). This highlights the importance of targeted analyses of AAA subgroups defined by relevance to clinical practice. *Correlations between baseline AAA diameter and future 12-month AAA growth*

Eighty-eight of the participants with small to moderate size AAAs (30-55mm) under surveillance were re-assessed at 12 months. The median duration of follow-up for this group was 365 days (IQR: 343 to 381 days). The median progression rate over this period for this combined group of patients was 2.8% per year (IQR: 0% to 5.7% /year). There was an overall correlation between the baseline diameter of AAAs and the growth rate in the future 12 months (Fig 2-A, Spearman r = .30, P<.005).

When the participants were further stratified according to AAA diameter (30-39mm, 40-55mm), the median growth rate over the following 12-month period differed significantly between the small AAAs (30-39mm) and the moderate AAAs (40-55mm) (2.6% vs 4.2%, Mann-Whitney test P<.01). Further examination of the AAA subgroups showed no correlation between baseline diameter vs the future growth rate in either the moderate size AAAs (40-55mm) (Figure 2-B, Spearman r = .12, P=.39) or in the subgroup of small size AAAs (30-39mm (Figure 2-C, Spearman r = -.02, P =.9).

These observations suggest the significant correlation observed in Figure 2-A to be underpinned by the overall disparate growth rates observed between the groups of small (30-39mm) and moderate (40-55mm) AAAs. Whereas within these subgroups of small and moderate AAA the baseline diameter has no predictive value on its future growth rate. This evidence suggests that baseline diameter alone is not a useful predictor of future growth rates when patients are stratified into small and moderate size subgroups.

### Correlation between FMD and future AAA growth

During the same period of follow up, FMD at baseline correlated significantly with the *future* growth rate. This significant weak correlation was observed in both small and moderate AAAs (30-55mm). (Figure 2-D, Spearman r = -.35, P<.005). Within the subgroups, there was also a significant and moderate correlation between FMD and future 12-month growth in the moderate size AAAs (40-55mm) (Figure 2-E, Spearman r = -.40, P<.005). This evidence suggests FMD may be a predictive marker for future AAA growth, particularly in the moderate size AAAs.

### FMD deteriorates during AAA progression, and is improved by AAA surgery

FMD reduced significantly between assessments during AAA surveillance (from a median of 2.0% at baseline to 1.2% at follow up, Wilcoxon matched pairs signed rank test [WMPST] P=.004). FMD was measured in 50 patients prior to AAA surgery, and remeasured at 8-12 weeks after. FMD improved significantly after surgical repair (from 1.1% pre-op to 3.8% post-op, WMPST P<.0001), irrespective of the type of surgery performed (EVAR n=28; open surgical repair n=22) (Figure 3).

### NMD and AAA progression

For 76 patients, dilatation measurements were performed with pharmacological supplementation of nitroglycerin to assess the endothelium independent vasodilatory response (nitroglycerin mediated dilatation, NMD). This serves as a comparator for FMD mediated through the endogenous release of nitric oxide. There was no correlation between AAA diameter and NMD. NMD did not deteriorate under AAA surveillance.

### Discussion

In addition to being the largest prospectively recruited cohort to date that examines FMD in the context of human AAAs, the examples demonstrate these findings: FMD correlates with future growth in AAAs; FMD deteriorates during AAA progression; FMD improves after the surgical treatment of AAAs. These findings suggest FMD can be a novel biomarker of AAA progression. Biomarkers of AAA progression can have a significant impact on clinical practice in the era of personalised medicine.

The size of an AAA is conventionally regarded as a predictor of its subsequent rate of progression. This correlation may be observed with larger AAA diameters (e.g. 55 mm and larger) However, it is important to note that the assessment of associations between "baseline AAA diameter" versus "future AAA growth" is inherently confounded by the fact that these two variables are both derivative of AAA diameter and therefore are co-variates by default. Interestingly, within the moderate size (40-55mm) group, the diameter at baseline was no longer predictive of the future AAA growth but FMD was predictive. These findings suggest the utility of FMD as a potential biomarker of future AAA progression will be fruitful, particularly in the 40-55mm group.

The moderate size (40-55mm) AAA group may be important in terms of relevance to clinical practice because there may be a significant number of patients in this group who may benefit e.g. from a surgical intervention even though their AAAs have not reached the threshold surgical size (55mm).

The Examples also demonstrate the deterioration of FMD during the natural history of AAA surveillance, which is reversible by AAA surgery irrespective of the surgical techniques.

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

Features of the present invention are now described in the following numbered paragraphs:
1. Apparatus for determining an index of aneurysm future growth for a subject, the apparatus comprising: a data input to receive at least one index of endothelial function of a subject; at least one processor; and a memory comprising instructions executable by the at least one processor to cause the processor to: compare the or each index of endothelial function with at least one of: a respective reference value; and a further index of endothelial function for the subject; and determine the index of aneurysm future growth based on the comparison.
2. The apparatus as described in paragraph 1, wherein the or each index of endothelial function is indicative of at least one of: a flow-mediated vasodilation value, a flow-mediated constriction value, a concentration of one or more circulating biomarkers of endothelial function and a previous aneurysm growth pattern of the subject.
3. The apparatus as described in paragraph 2, wherein the circulating biomarkers of endothelial function are one or more of: an endothelin protein; a relaxin protein; a plurality of proteins and/or metabolites, whereby to form a circulating protein and/or metabolite profile; and a plurality of genes, whereby to form a genomic profile.
4. The apparatus as described in any of paragraphs 1 to 3, further comprising: a sensor device in data communication with the data input and configured to measure one or more of the at least one index of endothelial function.
5. The apparatus as described in paragraph 4, wherein the sensor device comprises a blood pressure cuff or biomarker measurement module to measure at least one of a flow-mediated vasodilation value and a flow-mediated constriction value.
6. The apparatus described in any of paragraphs 1 to 5, further comprising a data memory in data communication with the data input, the data memory storing one or more of the at least one index of endothelial function.
7. The apparatus as described in any of paragraphs 1 to 6, wherein, when the instructions cause the processor to compare the or each index of endothelial function with the further index of endothelial function, one or more of the at least one index of endothelial function is based on measurements taken at a first time and the further index of endothelial function is based on measurements taken at a second time different from the first time.
8. The apparatus as described in any of paragraphs 1 to 7, wherein, when the instructions cause the processor to compare the or each index of endothelial function comprises with the further index of endothelial function, an index type of one or more of the at least one index of endothelial function is different from an index type of the further index of endothelial function.
9. The apparatus as described in any paragraph dependent on paragraph 2, wherein, when one or more of the at least one index of endothelial function is indicative of the concentration of one or more circulating biomarkers, the apparatus further comprises a blood sample analysis module, and wherein the memory further comprises instructions to cause the processor to: analyse, in vitro using the blood sample analysis module, a sample derived from a blood sample of the subject; and determine the one or more of the at least one index of endothelial function based on the analysis of the sample.
10. The apparatus as described in any paragraph dependent on paragraph 2, wherein, when one or more of the at least one index of endothelial function is indicative of a flow-mediated vasodilation value, the memory further comprises instructions to cause the processor to: determine a dilatation value indicative of a vasodilation of a blood vessel as a result of an increase in fluid flow rate through the blood vessel; and determine the one or more of the at least one index of endothelial function based on the determination of the dilatation value.
11. The apparatus as described in any paragraph dependent on paragraph 2, wherein, when one or more of the at least one index of endothelial function is indicative of a flow-mediated constriction value, the memory further comprises instructions to cause the processor to: determine a constriction value indicative of a constriction of a blood vessel as a result of a decrease in fluid flow rate through the blood vessel; and determine the one or more of the at least one index of endothelial function based on the determination of the constriction value.
12. The apparatus described in any of paragraphs 1 to 11, wherein the memory further comprises instructions to cause the processor to store the index of aneurysm future growth in the memory or a further memory.
13. A method for determining an index of aneurysm future growth for a subject, the method comprising: receiving at least one index of endothelial function of the subject; comparing the or each index of endothelial function with at least one of: a respective reference value; and a further index of endothelial function of the subject; and determining the index of aneurysm future growth based on the comparison.
14. A computer-implemented method for determining an index of aneurysm future growth for a subject, the method comprising: a memory of a computer receiving at least one index of endothelial function of the subject; at least one processor of the computer reading from memory the at least one index of endothelial function; the at least one processor of the computer comparing the index of endothelial function with at least one of: a respective reference value; and a further index of endothelial function of the subject; and the at least one processor determining the index of aneurysm future growth based on the comparison.
15. The computer-implemented method of paragraph 14, further comprising storing the determined index of aneurysm future growth in the memory or a further memory.
16. A non-transitory machine-readable storage medium encoded with instructions executable by a processor, the machine-readable storage medium comprising instructions to: receive at least one index of endothelial function of a subject having an aneurysm; compare the index of endothelial function with at least one of a respective reference value and a further index of endothelial function of the subject; and determining an index of aneurysm future growth for the subject based on the comparison.

Further features of the present invention are also set out in the following numbered paragraphs.
1. A method for determining a risk factor indicative of predicted growth of an abdominal aortic aneurysm of a patient, the method comprising: receiving:a value representative of a size of the abdominal aortic artery; and a flow-mediated vasodilation or flow-mediated constriction value (FMD/C value) of an artery of the patient; and determining the risk factor indicative of predicted growth by evaluating the received values with those in an aneurysm risk model, the aneurysm risk model relating to a risk value indicative of predicted growth of an abdominal aortic aneurysm for a given value representative of the size of an abdominal aortic artery and a given FM D/C value.
2. The method described in paragraph 1 further comprising guiding a decision on a clinical intervention of the patient based on the determined risk factor.
3. The method as described in paragraph 2 wherein the clinical intervention is an invasive clinical intervention if the size of the abdominal aortic artery is between and including a range of 40 millimetres and 55 millimetres.
4. The method as described in paragraph 2, wherein the clinical intervention is a non-invasive or cognitive clinical intervention.
5. The method as described in any of paragraphs 1 to 4, wherein the method comprises receiving a value representative of a size of the abdominal aortic artery; and a flow-mediated vasodilation value (FMD value) of an artery of the patient.
6. The method as described in paragraph 5, wherein the FMD value is the FMD value of the brachial artery of a patient.
7. The method as described in paragraph 5 or 6, further comprising measuring the FMD value at the brachia! artery of the patient.
8. The method as described in paragraph 7, further comprising measuring the FMD value by means of continuously imaging the brachia! artery using ultrasound.
9. The method as described in any of paragraphs 1 to 8, wherein the aneurysm risk model is a look-up table.
10. The method as described in any of paragraphs 1 to 9, wherein the aneurysm risk model is generated based on:FMD/C measurements and the size of an abdominal aortic artery of a patient taken at a first time; and FMD/C measurements and the size of an abdominal aortic artery of a patient taken at a second time different from the first time.
11. The method as described in paragraph 10, wherein the aneurysm risk model is also generated on the basis of the value representative of a size of the abdominal aortic artery at the first time and the size of the abdominal aortic artery at the second time.
12. The method as described in any of paragraph 1 to 11 wherein the method is used to determine a risk factor indicative of predicted growth of an abdominal aortic aneurysm of a patient identified as having an abdominal aortic artery that is 35 to 55mm or 40 to 55 mm in size.
13. The method as described in any of paragraph 1 to 12 wherein the received value representative of a size of the abdominal aortic artery is representative of an abdominal aortic artery that is 35 to 55mm or 40 to 55 mm in size.
14. The method as described in any of paragraphs 1 to 13 wherein the given value representative of the size of an abdominal aortic artery of the aneurysm risk model is 35 to 55mm or 40 to 55 mm in size.
15. Apparatus for determining a risk factor indicative of predicted growth of an abdominal aortic aneurysm of a patient, the apparatus comprising: a data input to receive: a value representative of a size of an abdominal aortic artery of a patient; and a flow-mediated vasodilation or flow-mediated constriction value (FMD/C value) of an artery of the patient; at least one processor; and a memory comprising: an aneurysm risk model relating to a risk value indicative of predicted growth of an abdominal aortic aneurysm for a given value representative of the size of an abdominal aortic artery and a given FMD/C value; and instructions executable by the at least one processor to cause the processor to retrieve from the aneurysm risk model, using the received data inputs, a risk factor indicative of predicted growth of an abdominal aortic aneurysm.
16. The apparatus as described in paragraph 15, wherein the risk factor is subsequently usable to guide a decision on a clinical intervention of the patient.
17. The apparatus as described in paragraph 16 wherein the clinical intervention is an invasive clinical intervention if the size of the abdominal aortic artery is between and including a first range of 40 millimetres and 55 millimetres.
18. The apparatus as described in paragraph 16 wherein the clinical intervention is a non- invasive or cognitive clinical intervention.
19. The apparatus as described in any of the paragraphs 15 to 18, wherein the data input receives a value representative of a size of the abdominal aortic artery; and a flow-mediated vasodilation value (FMD value) of an artery of the patient.
20. The apparatus as described in paragraph 19, wherein the FMD value is an FMD value of a brachia! artery of the patient.
21. The apparatus as described in any of the paragraphs 15 to 20, further comprising: a sensor device in data communication with the data input and configured to measure the FMD value at the brachia! artery of the patient.
22. The apparatus as described in paragraph 21, wherein the sensor device measures the FMD value by means of continuously imaging the brachia! artery using ultrasound.
23. The apparatus as described in any of the paragraphs 15 to 22, wherein the aneurysm risk model is a look-up table.
24. The apparatus as described in any of the paragraphs 15 to 23, wherein the aneurysm risk model is generated based on: FMD/C measurements and the size of an abdominal aortic artery of a patient taken at a first time; and FMD/C measurements and the size of an abdominal aortic artery of a patient taken at a second time different from the first time.
25. The apparatus as described in paragraph 24, wherein the aneurysm risk model is also generated on the basis of the value representative of a size of the abdominal aortic artery at the first time and the size of the abdominal aortic artery at the second time.
26. A non-transitory machine-readable storage medium encoded with instructions executable by a processor for determining a risk factor indicative of predicted growth of an abdominal aortic aneurysm of a patient, the machine-readable storage medium comprising instructions to: receive: a value representative of a size of the abdominal aortic artery; and a flow-mediated vasodilation or flow-mediated constriction value (FMD/C value) of the patient determine the risk factor indicative of predicted growth by evaluating the received values with those in an aneurysm risk model, the aneurysm risk model relating to a risk value indicative of predicted growth of an abdominal aortic aneurysm for a given value representative of the size of an abdominal aortic artery and a given FM D/C value.
27 The non-transitory machine-readable storage medium as described in paragraph 26 further comprising instructions to guide a decision on a clinical intervention of the patient based on the determined risk factor.
28. The non-transitory machine-readable storage medium as described in paragraph 27 wherein the clinical intervention is an invasive clinical intervention if the size of the abdominal aortic artery is between and including a range of 40 millimetres and 55 millimetres.
29. The non-transitory machine-readable storage medium as described in paragraph 27, wherein the clinical intervention is a non-invasive or cognitive clinical intervention.
30. The non-transitory machine-readable storage medium as described in any one of paragraphs 26 to 29, which comprises instructions to receive a value representative of a size of the abdominal aortic artery; and a flow-mediated vasodilation, value (FMD value) of an artery of the patient.
31. The non-transitory machine-readable storage medium as described in paragraph 30, wherein the FMD value is an FMD value of a brachial artery of the patient.
32 The non-transitory machine-readable storage medium as described in paragraphs 30 or 31, comprising instructions to measure the FMD value at the brachial artery of the patient.
33. The non-transitory machine-readable storage medium as described in paragraph 32 further comprising instructions to measure the FMD value by means of continuously imaging the brachial artery using ultrasound.
34. The non-transitory machine-readable storage medium as described in any of paragraphs 25 to 33, wherein the aneurysm risk model is a look-up table.
35. The non-transitory machine-readable storage medium as described in any of paragraphs 25 to 34, wherein the aneurysm risk model is generated based on: FMD/C measurements and the size of an abdominal aortic artery of a patient taken at a first time; and FMD/C measurements and the size of an abdominal aortic artery of a patient taken at a second time different from the first time.
36. The non-transitory machine readable storage medium as described in paragraph 35, wherein the aneurysm risk model is also generated on the basis of the value representative of a size of the abdominal aortic artery at the first time and the size of the abdominal aortic artery at the second time.

## Claims

1. Apparatus for determining an index of aneurysm future growth for a subject, the apparatus comprising: a data input to receive at least one index of endothelial function of a subject; at least one processor; and a memory comprising instructions executable by the at least one processor to cause the processor to: compare the or each index of endothelial function with at least one of: a respective reference value; and a further index of endothelial function for the subject; and determine the index of aneurysm future growth based on the comparison; wherein the or each index of endothelial function is indicative of a concentration of one or more circulating biomarkers of endothelial function.

2. An apparatus as claimed in claim 1, wherein the circulating biomarkers of endothelial function are one or more of: an endothelin protein; a relaxin protein; a plurality of proteins and/or metabolites, whereby to form a circulating protein and/or metabolite profile; and a plurality of genes, whereby to form a genomic profile.

3. An apparatus as claimed in claim 1 or claim 2, wherein the circulating protein and/or metabolite profile comprises over 50 proteins and/or metabolites.

4. An apparatus as claimed in any preceding claim further comprising a data memory in data communication with the data input, the data memory storing one or more of the at least one index of endothelial function.

5. An apparatus as claimed in any preceding claim, wherein, when the instructions cause the processor to compare the or each index of endothelial function with the further index of endothelial function, one or more of the at least one index of endothelial function is based on measurements taken at a first time and the further index of endothelial function is based on measurements taken at a second time different from the first time.

6. An apparatus as claimed in any preceding claim, wherein, when the instructions cause the processor to compare the or each index of endothelial function comprises with the further index of endothelial function, an index type of one or more of the at least one index of endothelial function is different from an index type of the further index of endothelial function.

7. An apparatus as claimed in any preceding claim, wherein the apparatus further comprises a blood sample analysis module, and wherein the memory further comprises instructions to cause the processor to: analyse, in vitro using the blood sample analysis module, a sample derived from a blood sample of the subject; and determine the one or more of the at least one index of endothelial function based on the analysis of the sample.

8. An apparatus as claimed in any preceding claim, wherein the memory further comprises instructions to cause the processor to store the index of aneurysm future growth in the memory or a further memory.

9. A method for determining an index of aneurysm future growth for a subject, the method comprising: receiving at least one index of endothelial function of the subject; comparing the or each index of endothelial function with at least one of: a respective reference value; and a further index of endothelial function of the subject; and determining the index of aneurysm future growth based on the comparison; wherein the or each index of endothelial function is indicative of a concentration of one or more circulating biomarkers of endothelial function and a previous aneurysm growth pattern of the subject.

10. A method as claimed in claim 9, wherein the circulating biomarkers of endothelial function are one or more of: an endothelin protein; a relaxin protein; a plurality of proteins and/or metabolites, whereby to form a circulating protein and/or metabolite profile; and a plurality of genes, whereby to form a genomic profile; optionally wherein the circulating protein and/or metabolite profile comprises over 50 proteins and/or metabolites.

11. A computer-implemented method for determining an index of aneurysm future growth for a subject, the method comprising: a memory of a computer receiving at least one index of endothelial function of the subject; at least one processor of the computer reading from memory the at least one index of endothelial function; the at least one processor of the computer comparing the index of endothelial function with at least one of: a respective reference value; and a further index of endothelial function of the subject; and the at least one processor determining the index of aneurysm future growth based on the comparison; wherein the or each index of endothelial function is indicative of a concentration of one or more circulating biomarkers of endothelial function and a previous aneurysm growth pattern of the subject.

12. A method as claimed in claim 11, wherein the circulating biomarkers of endothelial function are one or more of: an endothelin protein; a relaxin protein; a plurality of proteins and/or metabolites, whereby to form a circulating protein and/or metabolite profile; and a plurality of genes, whereby to form a genomic profile.

13. A method as claimed in claim 11 or claim 12, further comprising storing the determined index of aneurysm future growth in the memory or a further memory.

14. A non-transitory machine-readable storage medium encoded with instructions executable by a processor, the machine-readable storage medium comprising instructions to: receive at least one index of endothelial function of a subject having an aneurysm; compare the index of endothelial function with at least one of a respective reference value and a further index of endothelial function of the subject; and determining an index of aneurysm future growth for the subject based on the comparison; wherein the or each index of endothelial function is indicative of a concentration of one or more circulating biomarkers of endothelial function and a previous aneurysm growth pattern of the subject.

15. A medium as claimed in claim 14 wherein the circulating biomarkers of endothelial function are one or more of: an endothelin protein; a relaxin protein; a plurality of proteins and/or metabolites, whereby to form a circulating protein and/or metabolite profile; and a plurality of genes, whereby to form a genomic profile.
